# EUROPEAN PATENT SPECIFICATION

(11) **EP 1 787 578 B1**
(45) Date of publication and mention of the grant of the patent: **22.06.2011**
(21) Application number: 05780483.3
(22) Date of filing: 16.08.2005
(51) Int. Cl.: A61B 1/04

(54) **OBSERVATION SYSTEM**
BEOBACHTUNGSSYSTEM
SYSTEME D'OBSERVATION

(30) Priority: 23.08.2004 JP 2004242073
(43) Date of publication of application: 23.05.2007
(73) Proprietor: Olympus Corporation, Tokyo 151-0072 (JP)
(72) Inventor: YAMAGUCHI, Seiji, 1920913 (JP); MIYAMOTO, Shinichi, 1920916 (JP); KAMEYA, Takayuki, 1910016 (JP)
(74) Representative: von Hellfeld, Axel
(86) International application number: PCT/JP2005/014933
(87) International publication number: WO 2006/022165

(56) References cited:
- WO-A-02/055126
- JP-A- 6 327 678
- JP-A- 8 215 142
- JP-A- 11 000 310
- JP-A- 62 000 192
- JP-A- 2001 128 923
- JP-A- 2001 128 923
- US-A1- 2004 082 883

## Description

### TECHNICAL FIELD

The present invention relates to an observation system that functions as an endoscope.

### BACKGROUND ART

In the field of medicine, operations using endoscopes are widely performed, and in recent years portable type endoscopes are widely used. These portable type endoscopes have small monitor devices and batteries or the like integrally or detachably provided, making them extremely easy to carry. Such portable endoscopes can be used in a wide variety of environments, for example enabling endoscope operations to be made easily by a house call on patients in home care and the like, and as such they offer a high level of convenience.

However, there are many kinds of operations, and it has been necessary to prepare and to bring along an endoscope having dedicated functions for each operation. Furthermore, in many cases, until the operation is actually carried out, it is not known which type of functions are required, and in such cases multiple types of endoscopes have had to be carried at one time. Moreover, in a general endoscope, a plurality of functions are often provided in a single endoscope so that in the case in which several types of endoscopes have been prepared, there is an overlap of functions between them. In other words, in the case where several types of endoscope are brought along at once, not only inconvenience for the endoscope user such as the doctor, but a functional redundancy is also created.
[Patent Document 1] Japanese Unexamined Patent Application, First Publication No. 2001-128923.
Patent Document 1 discloses an endoscope instrument comprising a group of insert units and a group of holder units. The group of insert units comprises plural kinds of different insert units with different forms, and the group of holder units comprises plural kinds of different holder units with different forms. In the endoscope, each insert unit in the group of insert units can be freely combined with/connected to each holder unit in the group of holder units.
US 2004/0082883 A1 refers to an ultrasound endoscope, wherein a rigid tip end section which is connected to an angle section at the fore distal end of an insertion instrument of an ultrasound endoscope is housed in a casing which can be split into a main casing and a separable head block to facilitate maintenance and service of internal component parts of the rigid tip end section. An ultrasound transducer is accommodated in a front side portion of the main casing, while endoscopic observation means including an illumination means and an optical image pickup means are fitted in an inclined wall rising obliquely upward on the rear side of ultrasound transducer. An outlet opening of a biopsy channel outlet passage is located between the ultrasound transducer and the endoscopic observation means. The main casing is adapted to accommodate the ultrasound transducer and its wiring, while the separable head block is adapted to accommodate at least part of component parts of the endoscopic observation means. The main casing and the separable head block are joined with each other through joint wall portions provided along split lines at the opposite lateral sides and at the front side thereof. A foremost one of angle rings of the angle section is detachably fitted on base end portions of the main casing and separable head block to retain these parts in a fixedly connected state.

### DISCLOSURE OF INVENTION

### [Problem to be Solved by the Invention]

As an endoscope to solve such problems, for example an endoscope disclosed in Patent Document 1 is known. This endoscope, by having the endoscope user select and combine one each of the most appropriate items from a group of insertion section units and a group of holder section units, is said to be able to satisfy different and various demands dependant on the size and direction of the target observation portion, the objective of the observation, or the environment in which the endoscope is to be used and so forth. However, the insertion section unit and holding section unit must be made to correspond one to the other in combining them, so that there are certain limitations on the level of freedom of combination. For this reason, it has been as difficult as ever to construct an endoscope that combines a single or several required functions from among many functions, to assure just a minimal requirement of inherent functions.

The present invention takes the above into consideration, and its object is to provide an observation system in which an observation unit having an inherent function can be combined at will with a holding device that holds it, and in which the function of an endoscope can be assured in a single form.

### [Means for Solving the Problem]

The present invention is an observation system as defined by independent claim 1.

Since it has this kind of construction, by changing the construction of the image information obtaining device or the image signal processing device, a plurality of observation units, each having an inherent function, can be obtained. Then, by assembling the one or plurality of observation units and the holding device, an observation system that functions as an endoscope with inherent functions can be constructed.

The transmission device may be a wireless transmitting and receiving device that can transmit the image signal wirelessly to a peripheral device, and that can receive various kinds of signals wirelessly from a peripheral device.

When wireless transmission and reception is thus enabled, the inconvenience of interposing a cord etc is done away with, so that image signals can be sent from the observation unit to a peripheral device, and signals can be sent from a peripheral device to the observation unit.

One or a plurality of the observation units are held inside the cylinder shaped body to obtain image information of the observation target via the window section.

Since the holding device is a cylinder shaped body, the observation unit can be easily attached and detached, and it can easily hold a plurality of observation units.
Also, bodily fluids such as blood do not become attached to the observation unit, so that cleaning between operations is made simpler. Therefore, in the case of home care, cleaning need not be carried out every time, so a high level of convenience can be ensured.

The observation unit may be an image capture unit provided with an image capture device that is adapted to image-capture an observed image of the observation target, as the image information obtaining device.

When the observation unit is an image capture unit, an observed image of an observation target such as a body cavity interior for example can be easily image-captured, and image information easily obtained.

The observation unit may be an ultrasound unit provided with an oscillator that is adapted to emit ultrasonic waves toward an observed portion of the observation target and to receive ultrasonic waves reflected from the observed portion, as the image information obtaining device.

When the observation unit is an ultrasound unit, a tomographic image of an observed portion such as a body cavity inner wall for example can be obtained as image information.

The holding device may be provided with a rotation driving device that is adpated to rotate the observation unit with respect to the holding device to vary an image information obtaining direction.

When the observation unit can be rotated to vary the image information obtaining direction, even if the holding device is fixed, image information of various directions can be obtained.

An auxiliary unit may be provided that is held together with the observation unit, by the holding device, and that is adapted to supply electric power to the observation unit.

By combining such an auxiliary unit, it becomes possible to drive the observation unit by the auxiliary unit, allowing it to be used for a long time.

### [Effect of the Invention]

By employing a construction such as described above, the observation system according to the present invention can provide an observation system in which an observation unit having an inherent function can be combined at will with a holding device that holds it, and in which the function of an endoscope can be assured in a single form.

### BRIEF DESCRIPTION OF DRAWINGS

[FIG. 1A] is a schematic side sectional view showing a basic embodiment of an observation unit used in an observation system according to a first embodiment of the present invention.
[FIG. 1B] is a schematic side sectional view showing a modified example of the observation unit shown in FIG. 1A.
[FIG. 1C] is a schematic side sectional view showing another modified example of the observation unit shown in FIG. 1A.
[FIG. 2A] is a schematic side sectional view showing still another modified example of the observation unit shown in FIG. 1A.
[FIG. 2B] is a schematic side sectional view showing still another modified example of the observation unit shown in FIG. 1A.
[FIG. 2C] is a schematic side sectional view showing still another modified example of the observation unit shown in FIG. 1A.
[FIG. 3A] is a schematic side sectional view showing still another modified example of the observation unit shown in FIG. 1A.
[FIG. 3B] is a schematic side sectional view showing still another modified example of the observation unit shown in FIG. 1A.
[FIG. 4A] is a schematic side sectional view showing still another modified example of the observation unit shown in FIG. 1A.
[FIG. 4B] is a schematic side sectional view showing still another modified example of the observation unit shown in FIG. 1A.
[FIG. 5A] is a schematic side sectional view showing an example of an ultrasound unit as an observation unit used in the observation system according to the first embodiment of the present invention.
[FIG. 5B] is a front view of the same ultrasound unit.
[FIG. 5C] is a schematic side sectional view showing another modified example of the ultrasound unit shown in FIG. 5A.
[FIG. 5D] is a front view of the same ultrasound unit.
[FIG. 5E] is a schematic side sectional view showing still another modified example of the ultrasound unit shown in FIG. 5A.
[FIG. 5F] is a front view of the same ultrasound unit.
[FIG. 6A] is a schematic structural view showing still another modified example of the observation units shown in FIG. 1A through FIG 4B.
[FIG. 6B] is a schematic structural view showing still another modified example of the ultrasound units shown in FIG. 5A through FIG 5F.
[FIG. 7A] is a schematic structural view showing one example of an observation system according to the first embodiment of the present invention.
[FIG. 7B] is a schematic structural view showing one example of an observation system according to the first embodiment of the present invention.
[FIG. 7C] is a schematic structural view showing one example of an observation system according to the first embodiment of the present invention.
[FIG. 8A] is a schematic structural view showing a modified example of the observation system shown in FIG. 7A through FIG. 7C.
[FIG. 8B] is a schematic structural view showing a modified example of the observation system shown in FIG. 7A through FIG. 7C.
[FIG. 8C] is a schematic structural view showing a modified example of the observation system shown in FIG. 7A through FIG. 7C.
[FIG. 9A] is a schematic structural view showing another modified example of the observation system shown in FIG. 7A through FIG. 7C.
[FIG. 9B] is a schematic structural view showing another modified example of the observation system shown in FIG. 7A through FIG. 7C.
[FIG. 10A] is a schematic structural view showing still another modified example of the observation system shown in FIG. 7A through FIG. 7C.
[FIG. 10B] is a schematic structural view showing still another modified example of the observation system shown in FIG. 7A through FIG. 7C.
[FIG. 11A] is a schematic structural view showing still another modified example of the observation system shown in FIG. 7A through FIG. 7C.
[FIG. 11B] is a schematic structural view showing still another modified example of the observation system shown in FIG. 7A through FIG. 7C.
[FIG. 12A] is a schematic structural view showing still another modified example of the observation system shown in FIG. 7A through FIG. 7C.
[FIG. 12B] is a schematic structural view showing still another modified example of the observation system shown in FIG. 7A through FIG. 7C.
[FIG. 13A] is a schematic structural view showing still another modified example of the observation system shown in FIG. 7A through FIG. 7C.
[FIG. 13B] is a schematic structural view showing still another modified example of the observation system shown in FIG. 7A through FIG. 7C.
[FIG. 14] is a perspective view showing an observation system according to a second embodiment of the present invention.
[FIG. 15A] is a perspective view showing the observation system according to the second embodiment of the present invention.
[FIG. 15B] is a partial side sectional view of the same observation system.
[FIG. 16A] is a perspective view showing an observation system according to a third exemplary embodiment useful for understanding the present invention.
[FIG. 16B] is a side view of the same ultrasound unit.
[FIG. 17] is a sectional side view of the same ultrasound unit.
[FIG. 18A] is a partial sectional side view showing a modified example of the observation system shown in FIG. 16A and FIG. 16B.
[FIG. 18B] is a partial sectional side view showing a modified example of the observation system shown in FIG. 16A and FIG. 16B.
[FIG. 19A] is a perspective view showing an image capture unit used in an observation system according to a fourth exemplary embodiment useful for understanding the present invention.
[FIG. 19B] is a partial sectional side view of the same image capture unit.
[FIG. 20A] is a perspective view showing a holder unit used in an observation system according to the fourth exemplary embodiment useful for understanding the present invention.
[FIG. 20B] is a side view of the same holder unit.
[FIG. 21] is a side sectional view showing an observation system according to the fourth exemplary embodiment useful for understanding the present invention.
[FIG. 22A] is a side sectional view showing a modified example of an observation system according to the fourth exemplary embodiment useful for understanding the present invention.
[FIG. 22B] is a perspective view of the same observation system.
[FIG. 22C] is a view on arrows X-X in FIG. 22B.
[FIG. 23] is a front view showing one example of a display device as a preferred peripheral device for use with the observation system according to the present invention.
[FIG. 24A] is a front view showing a modified example of the display device shown in FIG. 23.
[FIG. 24B] is a front view showing a modified example of the display device shown in FIG. 23.

### [Description of the Reference Symbols]

E1, E2, E3, E4, E5, E6, E7 Hard endoscope (Observation system)
E11 Observation System
E12, E15, E16 Hard endoscope (Observation system)
1A,1B, 1C, 1D, 1E, 1F,1G, 1H, 1I, 1J, 1K Image capture unit (observation unit)
2A, 2B, 2C, 2D Ultrasound unit (observation unit)
3 Auxiliary unit
5A, 5B, 5C, 5D, 5E, 5F Hard tube (holding device)
7 Holding member (holding device)
9A, 9B Holding member (holding device)
12A, 12B, 12C, 12D, 12E, 12F Image capture element (image information obtaining device)
13 CCU (image signal processing device)
14 Wireless circuit (transmission device, wireless transmission and reception device)
21A Array oscillator (oscillator, image information obtaining device)
21B, 21C Array oscillator (oscillator, image information obtaining device)
23 Control circuit (image signal processing device)
51, 52, 53, 54 Window section
63m Motor (Rotation driving device)
64 Rotating member (Rotation driving device)
81 Motor (Rotation driving device)
92 Motor (Rotation driving device)

### BEST MODE FOR CARRYING OUT THE INVENTION

Embodiments of the present invention and exemplary embodiments useful for understanding the present invention are described below, using the drawings.

### [First Embodiment]

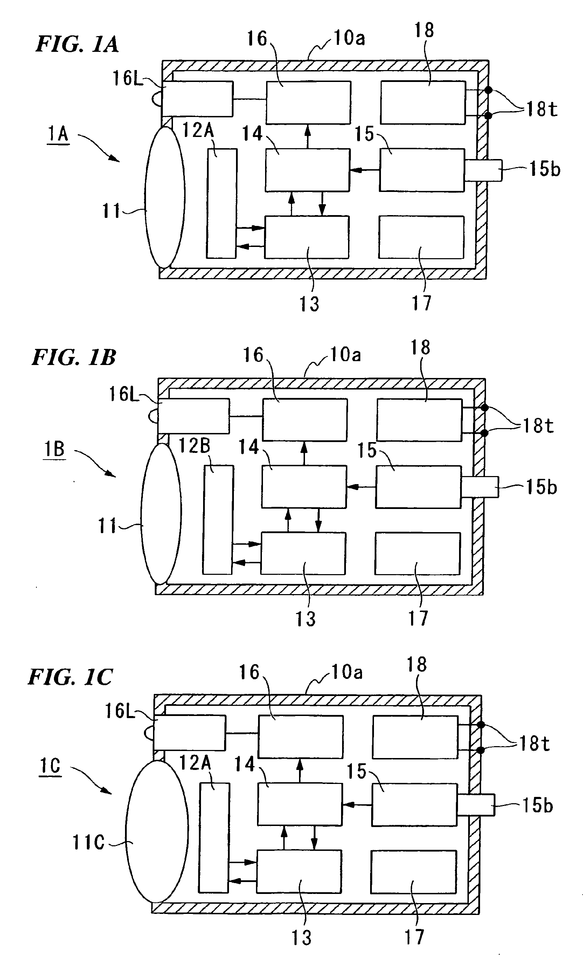

Firstly, a first embodiment will be described using FIG. 1A through FIG. 13B. In the present embodiment, an example is described in which a hard endoscope to be inserted inside the body cavity of a patient (observation target) is constructed as an observation system using an image capture unit or an ultrasound unit as an observation unit, and a hard tube as a holding device.

First is a description of the image capture unit. This image capture unit captures images of an observed image such as of an inner wall of a body cavity of a patient, to obtain image data.

A basic embodiment of the image capture unit used in the present embodiment is shown in FIG. 1A. This image capture unit (observation unit) 1A is constructed with an optical system, an image capture element (image information obtaining device) 12A, a CCU (Camera Control Unit) (image signal processing device) 13, a wireless circuit (transmission device, wireless transmission and reception device) 14, a drive switch section 15, a lighting circuit 16, a light emitting element 16L, a battery 17, and an auxiliary power supply circuit 18, all provided integrally inside a case 10a.

The case 10a is a substantially circular column shape or polygonal shape when seen from the front, and has a lens 11 and a light emitting element 16L as an optical system, provided in the front face (forward face) side (left side in the drawing) thereof. To the rear side of the lens 11, there is provided an image capture element (image information obtaining device) 12A, including a CCD (charge coupled device) or the like, by which the observed image from the lens 11 is imaged. This image capture element 12A captures the imaged observation image to obtain the observed image as image information, then converts this information to an image signal and outputs it to a CCU 13. As an optical system, an aperture, shutter or the like (not shown in the drawing) may be appropriately provided between the lens 11 and the image capture element 12A, or alternatively, such mechanisms may be omitted and continuous image data may be processed electronically by the CCU 13.

The CCU 13 is a control circuit for comprehensively controlling the operations of each of the components inside the image capture unit 1A, and also functions as an image signal processing device for processing the image signal that is outputted from the image capture element 12A. After image signal processing has been carried out on it by the CCU 13, the image signal is outputted to the wireless circuit 14.
The wireless circuit (transmission device, transmission and reception device) 14 carries out wireless transmission to peripheral devices of the image signal that is inputted from the CCU 13, and also wireless reception of instruction signals from peripheral devices, which it then outputs to the CCU 13. The CCU 13 receives these instruction signals and comprehensively controls the operations of the image capture unit 1A.

A drive switch unit 15 performs ON/OFF switching of a driving power supply of the image capture unit 1A, and is provided with a push-button switch 15b that passes through and projects out from the back side (reverse side) (left side in the drawing) of the case 10a. On pressing this push-button switch 15b toward the front face side, the driving power supply is turned ON and the image capture system 1A is driven, and when the pressure is removed, the driving power supply is turned OFF and the image capture system is stopped.

The light emitting element 16L includes an LED (Light Emitting Diode) or the like, and is provided in the vicinity of the lens 11 to shine visible light toward an observed portion. The lighting circuit 16 adjusts the light emission amount of, and switches ON and OFF, the light emitting element 16L according to a control signal from the CCU 13.
A battery 17 is housed replaceably inside the case 10a, and supplies electric power to each of the components of the image capture unit 1A. A secondary battery that can be repeatedly recharged and used, is used for this battery 17.
The auxiliary power supply circuit 18 is for receiving an external power supply apart from the battery 17, that is, from outside of the image capture unit 1A, and is provided with a terminal 18t that is electrically connected to the outside.

If the image capture unit 1A of such a construction is used, wireless remote control by a peripheral device such as a controller enables the observation image in front of the image capture unit 1A to be image-captured, and the captured image to be image-processed and then wirelessly transmitted to a peripheral device and then displayed on a display apparatus such as a monitor as a peripheral device.

Image capture units 1B through 1K, being modified examples of the image capture unit 1A, and ultrasound units 2A through 2D are described below. These image capture units 1 B through 1 K and ultrasound units 2A through 2D are units each having inherent functions that differ from that of the image capture unit 1A. In the description below, components that are the same as those in the image capture unit 1A are denoted by the same reference symbols, and detailed description thereof is omitted.

An image capture unit (observation unit) 1B shown in FIG. 1B has a high resolution image capture function, and in place of the image capture element 12A in the image capture unit 1A, an image capture element (image information obtaining device) 12B with more pixels is provided. An image capture unit (observation unit) 1C shown in FIG. 1C has a wide angle image capture function, and in place of the standard lens in the image capture unit 1A, a wide angle lens 11C is provided.

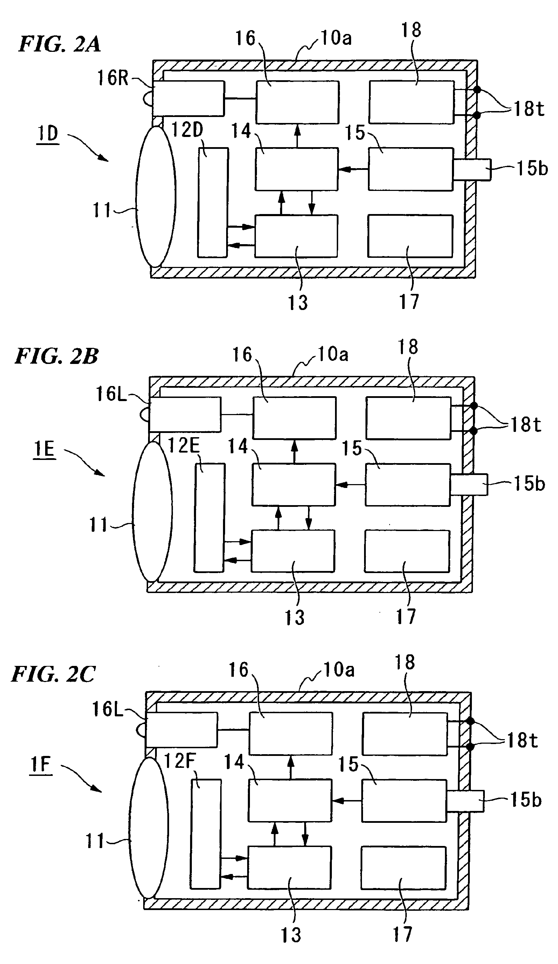

An image capture unit (observation unit) 1D shown in FIG. 2A has an infrared light image capture function, and in place of the image capture element 12A in the image capture unit 1A, an image capture element (image information obtaining device) 12D that is suited to receiving infrared light is provided, while in place of the light emitting element 16L, a light emitting element 16R that emits infrared light is provided. An image capture unit (observation unit) 1E shown in FIG. 2B has a fluorescent light image capture function, and in place of the image capture element 12A in the image capture unit 1A, an image capture element (image information obtaining device) 12E that is suited to receiving fluorescent light is provided. An image capture unit (observation unit) 1F shown in FIG. 2C has a function to enable stereo image capture, and in place of the image capture element 12A in the image capture unit 1A, an image capture element (image information obtaining device) 12F that is suited to stereo image capture is provided.

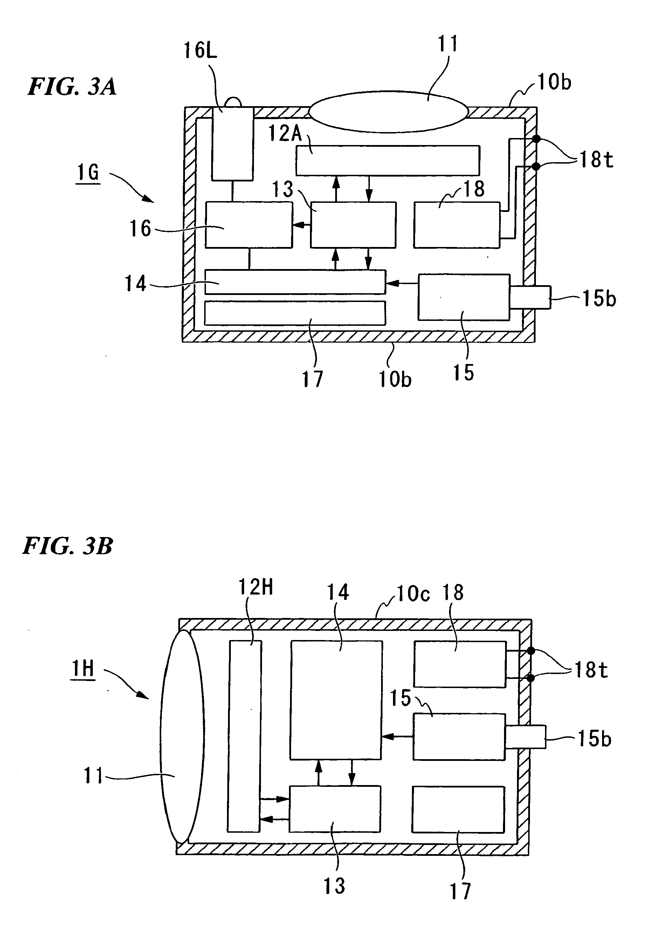

An image capture unit (observation unit) 1G shown in FIG. 3A is constructed with an optical system such as the lens 11, an image capture element 12A, a CCU 13, a wireless circuit 14, a drive switch unit 15, a lighting circuit 16, a light emitting element 16L, a battery 17, and an auxiliary power supply circuit 18, all integrally provided inside a case 10b. The case 10b is of substantially the same shape and construction as that of the case 10a, with the only point of difference being that the lens 11 and the light emitting element 16L are provided on the side face side. Also, each of the components inside the case 10b is disposed to fit the arrangement position of the lens 11 and the light emitting element 16L. That is to say, the image capture unit 1G has the image capture direction (image information obtaining direction) of the image capture unit 1A changed to the side direction.

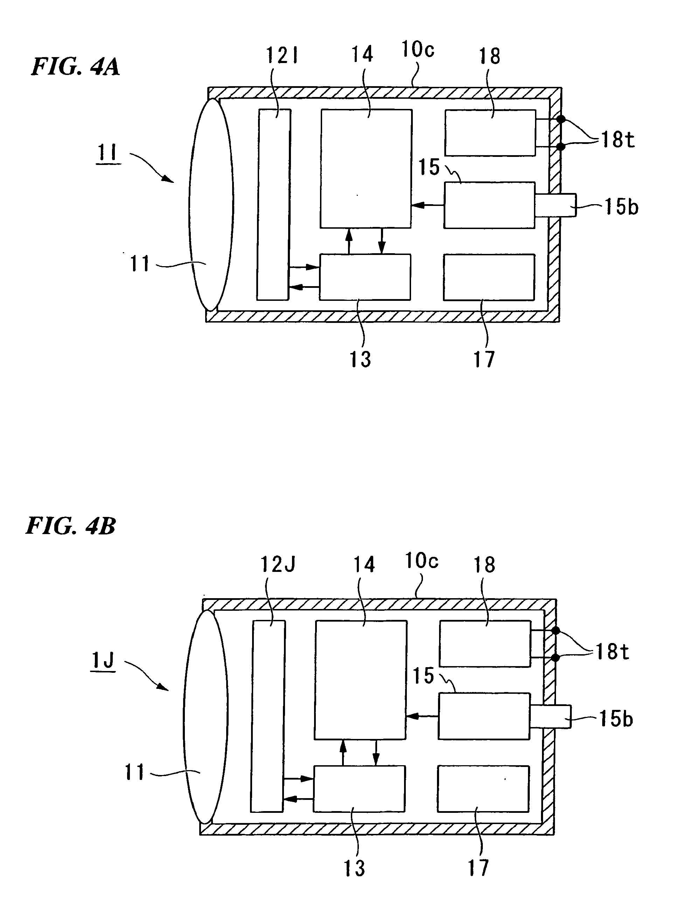

Next, each of the modified examples shown in FIG. 3B, FIG. 4A and FIG. 4B omits the light emitting devices such as the lighting circuit 16, and the light emitting elements 16L and 16R that were provided in the above image capture units 1A through 1 F, and has a simple construction.

An image capture unit (observation unit) 1H shown in FIG. 3B has a high resolution image capture function, and is constructed with an optical system such as the lens 11, an image capture element 12H, a CCU 13, a wireless circuit 14, a drive switch unit 15, a battery 17, and an auxiliary power supply circuit 18, all integrally provided inside a case 10c. The image capture element 12H is similar to the image capture element 12B of the image capture unit 1 B, in that it has more pixels.

An image capture unit (observation unit) 1I shown in FIG. 4A has an infrared light image capture function, and in place of the image capture element 12H in the image capture unit 1H, an image capture element (image information obtaining device) 12I that is suited to receiving infrared light is provided. This image capture element 12I is of a similar construction to the image capture element 12D in the above image capture unit 1D. Furthermore, the image capture unit (observation unit) 1J shown in FIG. 4B has a higher resolution infrared light image capture function, and in place of the image capture element I in the image capture unit 1I, an image capture element 12J that is suited to receiving infrared light and that has more pixels is provided.

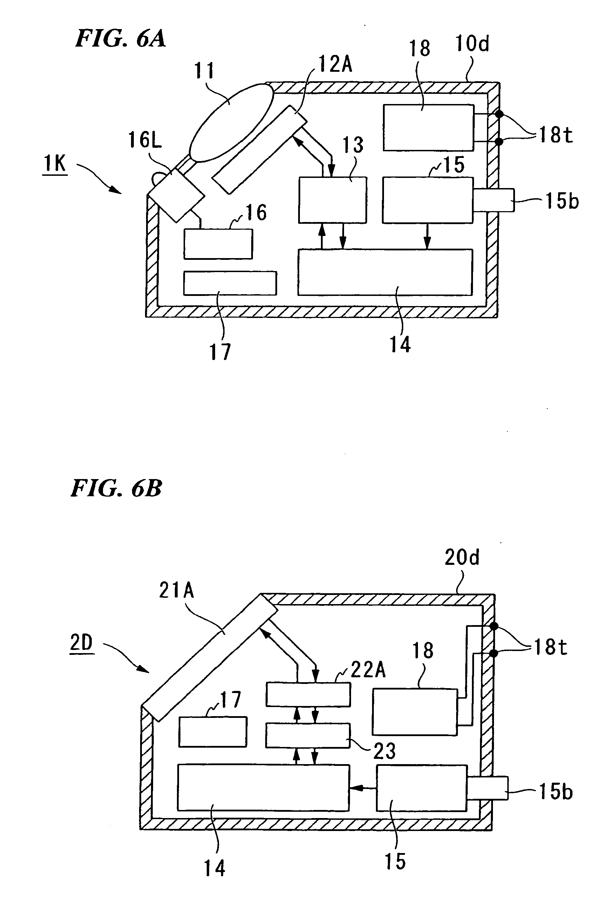

Moreover, an image capture unit (observation unit) 1K shown in FIG. 6A is constructed with an optical system such as the lens 11, an image capture element 12A, a CCU 13, a wireless circuit 14, a drive switch unit 15, a lighting circuit 16, a light emitting element 16L, a battery 17, and an auxiliary power supply circuit 18, all integrally provided inside a case 10d. The case 10d is of substantially the same shape and construction as that of the case 10a, with the only point of difference being that the lens 11 and the light emitting element 16L are provided on an incline running from the front face side to the side face side. Also, each of the components inside the case 10d is disposed to fit the arrangement position of the lens 11 and the light emitting element 16L. That is to say, the image capture unit 1K has the image capture direction of the image capture unit 1A changed to the forward-diagonal direction.

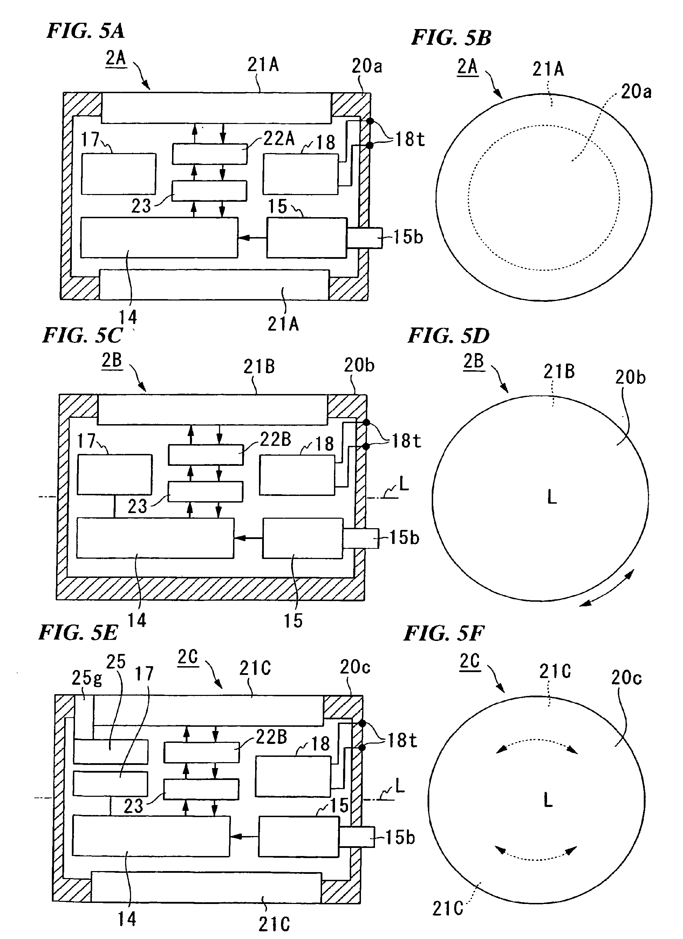

Next, the ultrasound unit used in the present embodiment will be described using FIGS. 5A through 5F, and FIG. 6B. These ultrasound units emit ultrasonic waves toward an observed portion of the inner wall of a body cavity of a patient and such, and receive the ultrasonic waves that are reflected from this observed portion to obtain image information.

The ultrasound unit (observation unit) 2A shown in FIG. 5A and FIG. 5B is constructed with a wireless circuit 14, a drive switch unit 15, a battery 17, an auxiliary power supply circuit 18, an array oscillator (oscillator, image information obtaining device) 21A, an oscillator driver 22A, and a control circuit 23, all integrally provided inside a case 20a.

The case 20a is a substantially circular column shape or polygonal shape when seen from the front, and is provided with an array oscillator 21 A all around the circumference of the side face thereof. The oscillator driver 22A drives the array oscillator 21A according to a control signal from the control circuit 23. The array oscillator 21A is driven by the oscillator driver 22A to emit ultrasonic waves toward the observed portion of the inner wall of a body cavity. The array oscillator 21A then receives the signal of the ultrasonic waves reflected from the observed portion to obtain image information such as a tomographic image of the internal tissue of the body cavity, and the array oscillator 21A converts the obtained image information into an image signal to be outputted to the control circuit 23.

The control circuit 23 is a circuit for comprehensively controlling the operations of each of the components inside the ultrasound unit 2A, and also functions as an image signal processing device for processing the image signal that is outputted from the array oscillator 21A. After image signal processing has been carried out on it by the control circuit 23, the image signal is outputted to the wireless circuit 14 to be transmitted wirelessly to the outside. Furthermore, instruction signals that the wireless circuit 14 receives wirelessly from the outside are outputted to the control circuit. The control circuit 23 receives these instruction signals and comprehensively controls the operation of the image capture unit 2A.

In this ultrasound unit 2A, the array oscillator 21A is provided all around the circumference of the side face of the case 20A, and can transmit and receive ultrasonic waves around 360°, and can therefore perform progressive scanning of a lumen such as the digestive canal for example, and can easily obtain a tomographic image or the like of the entire circumference thereof.

The ultrasound unit (observation unit) 2B shown in FIG. 5C and FIG. 5D is constructed with a wireless circuit 14, a drive switch unit 15, a battery 17, an auxiliary power supply circuit 18, an array oscillator (oscillator, image information obtaining device) 21B, an oscillator driver 22B, and a control circuit 23, all integrally provided inside a case 20b.

The case 20b is substantially the same as the above case 20a, the only point of difference being that the arrangement position of the oscillator is different. On one portion of the side face side of this case 20b there is provided an array oscillator 21B. The oscillator driver 22B drives the array oscillator 21B according to a control signal from the control circuit 23. The array oscillator 21B is driven by the oscillator driver 22A to emit ultrasonic waves toward an observed portion. This array oscillator 21B has fewer oscillator elements compared to the above array oscillator 21A, and therefore oscillator control can be simplified and it can be produced more cheaply. The array oscillator 21A then receives the signal of the ultrasonic waves reflected from the observed portion to obtain image information such as a tomographic image of the internal tissue of the body cavity, and the array oscillator 21A converts the obtained image information into an image signal to be output to the control circuit 23.

When using this ultrasound unit 2B it is preferable that an external rotation driving device rotates the ultrasound unit 2B around an axis L while obtaining the image information. By so doing, ultrasonic waves can be transmitted and received around the entire circumference (360°) of the ultrasound unit 2B, so that a tomographic image or the like of a lumen such as a digestive canal for example can be obtained around its entire circumference.

The ultrasound unit (observation unit) 2C shown in FIG. 5E and FIG. 5F is constructed with a wireless circuit 14, a drive switch unit 15, a battery 17, an auxiliary power supply circuit 18, an array oscillator (oscillator, image information obtaining device) 21C, an oscillator driver 22B, a control circuit 23, and a motor 25, all integrally provided inside a case 20c.

The case 20c is substantially the same as the above case 20a, the only point of difference being that the oscillator is rotatably supported. On one portion of the side face side of this case 20c, an array oscillator 21C similar to the above array oscillator 21B is supported so as to be rotatable around the axis L. This array oscillator 21C is rotation driven via a reduction gear 25g or the like, by the motor 25. The motor 25 is controlled by a control signal from the control circuit 23.

When using this ultrasound unit 2B, the motor 25 can rotate the array oscillator 21C around the axis L in relation to the case 20c while the image information is obtained. Therefore, ultrasonic waves can be transmitted and received around the entire circumference (360°) of the ultrasound unit 2C, so that, without requiring an external rotation driving device, a tomographic image or the like of a lumen such as a digestive canal for example can be obtained around its entire circumference.

Moreover, the ultrasound unit (observation unit) 2D shown in FIG. 6B is constructed with a wireless circuit 14, a drive switch unit 15, a battery 17, an auxiliary power supply circuit 18, an array oscillator (oscillator, image information obtaining device) 21A, an oscillator driver 22A, and a control circuit 23, all integrally provided inside a case 20d.

The case 20d is of substantially the same shape and construction as that of the case 20a, with the only point of difference being that the array oscillator 21A is provided on an incline running from the front face side to the side face side. Also, each of the components inside the case 20d is disposed to fit the arrangement position of the array oscillator 21A. That is to say, this ultrasound unit 2D has the ultrasound emitting and receiving direction (image information obtaining direction) of the ultrasound unit 2A changed to the forward diagonal direction. A wider area can be scanned by using a convex type oscillator (not shown in the drawing) in place of the array oscillator 21A.

In the ultrasound units 2B through 2D, if a construction that allows the ultrasonic wave output to be increased and focused into an extremely narrow range to pinpoint a particular place on the inner wall of a body cavity or the like, it is possible to destroy a lesion portion such as a tumor or ulcer, or a concretion. That is to say, in this case, the unit may have a function not only as an observation unit, but also as a therapeutic unit.

For each of the above described observation units (image capture units 1A through 1K, and ultrasound units 2A through 2D), it is preferable that an identifier inherent to each observation unit be provided.

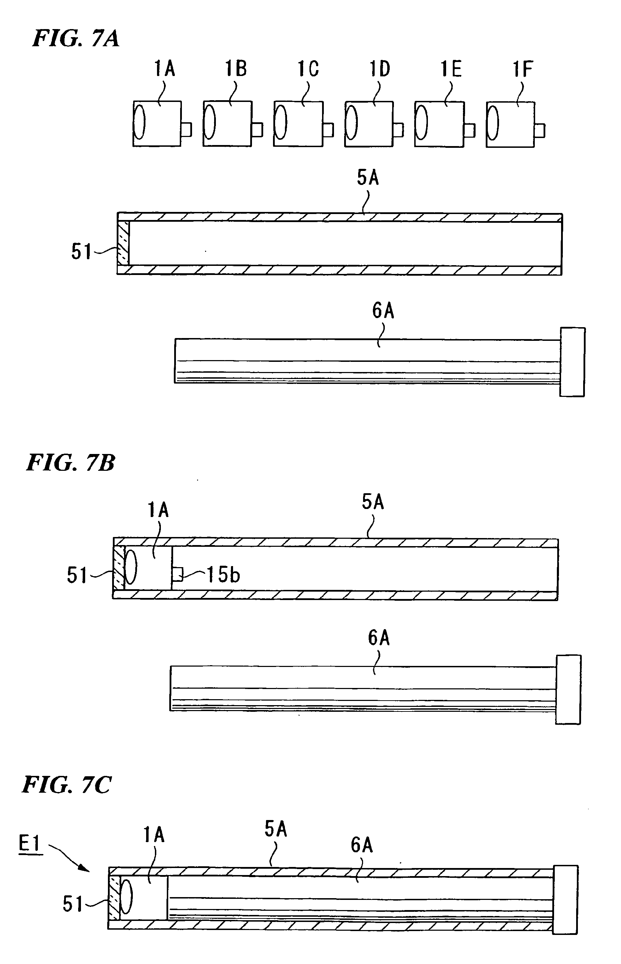

Next, an example construction of an endoscope that uses the above described image capture units 1A through 1K or ultrasound units 2A through 2D, is described using FIG. 7A through FIG. 13B. In each of these drawings, in order to show the image capture direction or ultrasound emitting direction, an approximate position of a lens or oscillator is schematically shown.

In FIG. 7A through FIG. 7C, an assembly example of a hard endoscope (observation system) E1 that preferably uses any one of the image capture units 1A through 1F is shown.
As shown in FIG. 7A, the endoscope is prepared with at least one of the image capture units 1A through 1F, a hard tube 5A, and an insertion member 6A. The hard tube 5A has a substantially circular cylinder shaped or substantially polygonal cylinder shaped cylindrical body, with a tip end side (left side in the drawing) which is to be inserted into a body cavity, having a closed end, and the tip end face section (front face section) thereof having a window section 51 including a transparent member formed thereon. Also, the insertion member 6A forms a substantially circular cylinder shape or polygonal cylinder shape having an outer diameter that corresponds to the inner diameter of the hard tube 5A, and is inserted into the hard tube 5A to position and fix the image capture unit inside the hard tube 5A.

Then, as shown in FIG. 7B, any one of the image capture units 1A through 1F (image capture unit 1A in the example of this drawing) is inserted inside the hard tube 5A. At this time, from the backward side of the image capture unit 1A, the push button switch 15b is protruding. Then, when the insertion member 6A is inserted inside the hard tube 5A, the front face of this insertion member 6A presses on the push-button switch 15b, so that the image capture unit 1A is driven and the image capture unit 1A is positioned and fixed inside the hard tube 5A in the forward side. Thus, as shown in FIG. 7C the image capture unit 1A, the hard tube 5A and the insertion member 6A construct a hard endoscope E1. In this way, by wireless remote operation of a peripheral device such as a controller, the image capture unit 1A can capture images of the observed image through the window section 51, to obtain image information in front of the hard endoscope E1. Then, after the captured image has been image-processed, it can be wirelessly transmitted to a peripheral device.

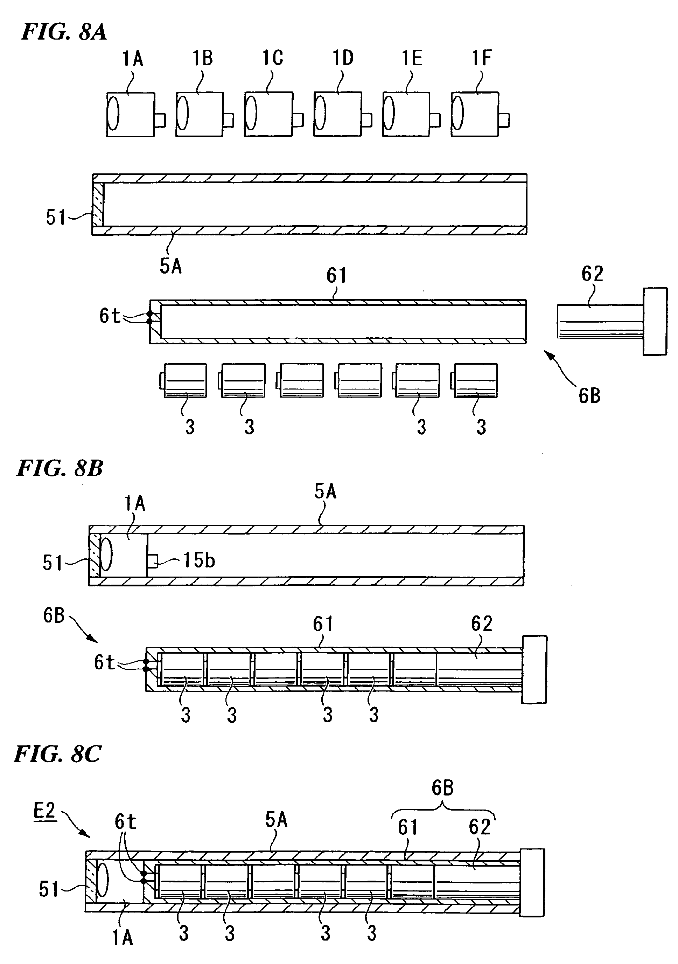

In FIG. 8A through FIG. 8C, a modified example of the above described hard endoscope E1 is shown as an assembly example of a hard endoscope (observation system) E2. In this example, an external battery unit of the image capture unit is provided as an auxiliary unit 3, and is configured to be able to supply electric power to the image capture unit.
FIG. 8A shows an insertion member 6B, which is the above described insertion member 6A made able to have the auxiliary unit 3 attached internally, being constructed from a cylinder-shaped body 61 of a bottomed-cylinder-shape, with the auxiliary unit 3 inserted thereinside, and a lid section 62 which acts as a lid to the back side of the cylinder-shaped body 61. On the tip end side of the cylinder shaped body 61 is formed a terminal 6t that electrically connects with the image capture unit terminal 18t (omitted from the drawing here) to enable supply of electric power from the auxiliary unit 3.

As shown in FIG. 8A, the endoscope is prepared with at least one of the image capture units 1A through 1F, a hard tube 5A, and an insertion member 6B. Then, as shown in FIG. 8B, any one of the image capture units 1A through 1F (image capture unit 1A in the example of this drawing) is inserted inside the hard tube 5A, and, as shown in FIG. 8C, the insertion member 6B is inserted inside the hard tube 5A. Thus, the hard endoscope E2 with the image capture unit 1A driven and the image capture unit 1A positioned and fixed in the forward side inside the hard tube 5A and the terminal 6t and the terminal 18t electrically connected is constructed. Accordingly, the image capture unit 1A is able to be driven by electric power from the auxiliary unit 3 as well as from the battery 17 inside the image capture unit 1A, enabling it to be used for a long time.

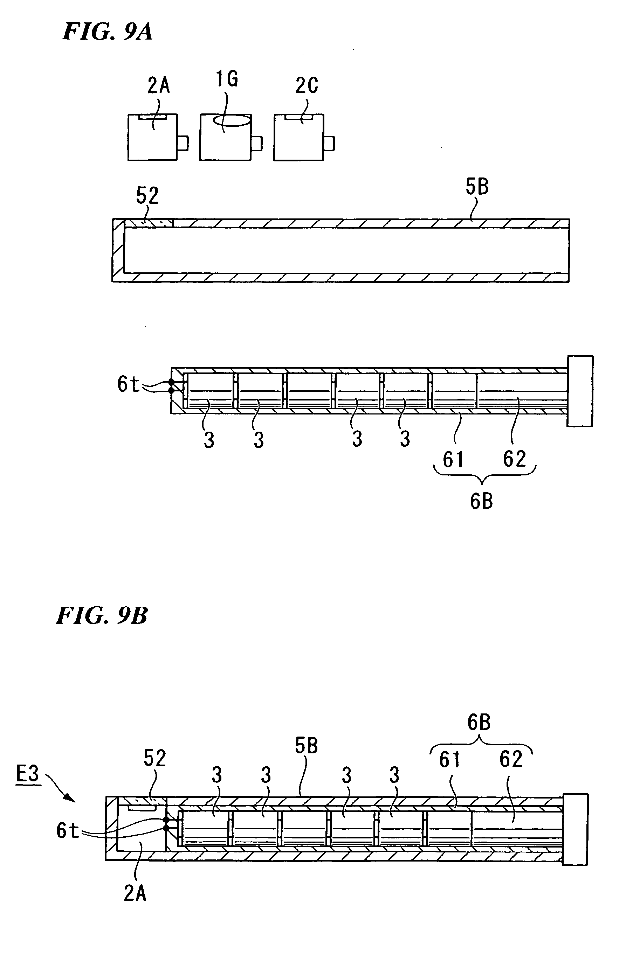

In FIG. 9A and FIG. 9B, an assembly example of a hard endoscope (observation system) E3 that preferably uses any one of the image capture unit 1G or the ultrasound units 2A and 2C is shown.
As shown in FIG. 9A, the endoscope is prepared with at least one of the image capture unit 1G or the ultrasound units 2A and 2C, a hard tube 5B, and an insertion member 6B. The hard tube 5B used here, is of similar construction to the above described hard tube 5A except that on the side face portion of the tip end side there is formed a window section 52 formed from a transparent member.

As shown in FIG. 9B, any one of the image capture unit 1G or the ultrasound units 2A and 2C (ultrasound unit 2A in the example of this drawing) is inserted inside the hard tube 5B, and the insertion member 6B is inserted inside the hard tube 5B. Thus, the hard endoscope E2 with the ultrasound unit 2A driven and the ultrasound unit 2A positioned and fixed in the forward side inside the hard tube 5B and the terminal 6t and the terminal 18t electrically connected is constructed. In this way, the hard endoscope E3 can obtain image information for the side direction of its tip end side.

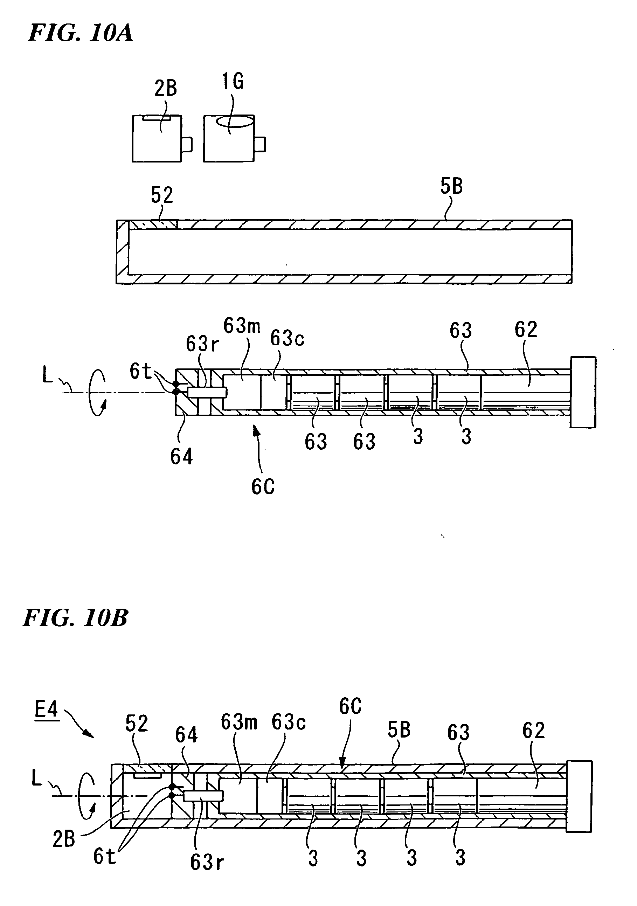

In FIG. 10A and FIG. 10B, a modified example of the above described hard endoscope E3 is shown as an assembly example of a hard endoscope (observation system) E4. In this example, the construction is one in which the image capture unit or ultrasound unit are rotation driven in the circumferential direction so that the image information obtaining direction can be changed, and any one of the image capture unit 1G or the ultrasound unit 2B is preferably used.
FIG. 10A shows an insertion member 6C, being constructed from a cylinder-shaped body 63 of a bottomed-cylinder-shape, with the auxiliary unit 3 inserted thereinside, and a lid section 62 which acts as a lid to the back side of the cylinder-shaped body 63, and a rotating member 64 that rotates the image capture unit or ultrasound unit integrally about the axis L. In the forward side inside the cylinder-shaped body 63 are housed a motor 63m that rotates the rotating member 64 about the axis L via a rotation shaft 63r, and a drive circuit 63c that performs drive control of this motor 63m. The drive circuit 63c and the motor 63m are both driven by electric power from the auxiliary unit 3. On the tip end side of the rotating member 64, there is formed a terminal 6t that electrically connects with the terminal 18t of the image capture unit or the ultrasound unit (omitted from the drawing here) to enable supply of electric power from the auxiliary unit 3.

As shown in FIG. 10A, the endoscope is prepared with at least one of the image capture unit 1G or the ultrasound unit 2B, a hard tube 5B, and an insertion member 6C. Then, as shown in FIG. 10B, any one of the image capture unit 1G or the ultrasound unit 2B (ultrasound unit 2B in the example of this drawing) is inserted inside the hard tube 5B, and the insertion member 6C is inserted inside the hard tube 5B. Thus, the hard endoscope E4 with the ultrasound unit 2B driven, and the ultrasound unit 2A pressed by the rotating member 64 to be integrated with the rotating member 64, and the terminal 6t and the terminal 18t electrically connected is constructed . Then, by rotation driving the motor 63m to rotate the rotating member 64 and the ultrasound unit 2B with respect to the hard tube 5B around the axis L, the side direction image information for the tip end side of the hard endoscope E4 can be obtained around almost the entire circumference.

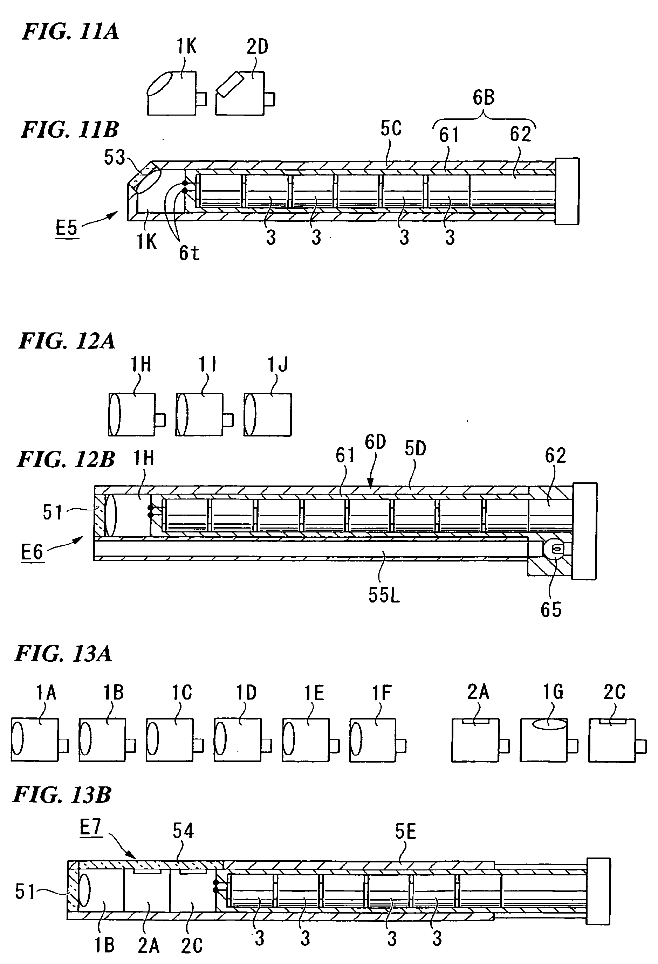

In FIG. 11A and FIG. 11B, an assembly example of a hard endoscope (observation system) E5 that preferably uses any one of the image capture unit 1K or the ultrasound units 2D is shown.
As shown in FIG. 11A and 11B, the endoscope is prepared with at least one of the image capture unit 1K or the ultrasound unit 2D, a hard tube 5C, and the insertion member 6B. The hard tube 5C used here, is of a similar construction to the above described hard tube 5A except that on the side face portion of the tip end side there is formed a window section 53 formed from a transparent member.

As shown in FIG. 11B, any one of the image capture unit 1K or the ultrasound unit 2D (image capture unit 1K in the example of this drawing) is inserted inside the hard tube 5C, and the insertion member 6B is inserted inside the hard tube 5C. Thus, a hard endoscope E5 with the image capture unit 1K driven, and the image capture unit 1K positioned and fixed in the forward side inside the hard tube 5C, and the terminal 6t and the terminal 18t electrically connected is constructed. In this way, the hard endoscope E5 can obtain image information of its forward diagonal direction.

In FIG. 12A and FIG. 12B, an assembly example of a hard endoscope (observation system) E6 that preferably uses any one of the image capture unit 1H through 1J, that is, image capture units that do not have a light emitting device, is shown. In this example, the construction is such that a light emitting device is provided externally to the image capture unit so that visible light or infrared light can be emitted forward for image capturing.

The hard tube 5D shown in FIG. 12B is constructed as the above described hard tube 5A with an additional light guide 55L. Moreover, the insertion member 6D is constructed as the above described insertion member 6B with an additional light source 65. For the light source 65, depending on which of the image capture units 1H through 1J is to be used, either a visible light emitting source or an infrared light emitting source may be used as appropriate.

As shown in FIG. 12B, any one of the image capture units 1H through 1J (image capture unit 1H in the example of this drawing) is inserted inside the hard tube 5D, and the insertion member 6D is inserted inside the hard tube 5D. Thus, the image capture unit 1H, the hard tube 5D and the insertion member 6D construct a hard endoscope E6. In this hard endoscope E6, the visible or infrared light emitted from the light source 65 passes through the light guide 55L to illuminate the observed portion from the tip end side, so that the image capture units 1H through 1J can be used to obtain image information in the forward direction.

In FIG. 13A and FIG. 13B, a hard endoscope (observation system) E7 that can use a image capture unit and an ultrasound unit in combination is shown as an assembly example.
As shown in FIG. 13A and 13B, the endoscope is prepared with a plurality from among the image capture units 1A through 1G and 1F and the ultrasound units 2A and 2C, a hard tube 5E, and the insertion member 6B. The hard tube 5E used here, is of similar construction to the above described hard tube 5A except that on the side face portion of the tip end side there is formed a window section 54 formed from a transparent member.

As shown in FIG. 13B, a plurality from among the image capture units 1A through 1G and 1F or the ultrasound units 2A and 2C (the three units, image capture unit 1A and ultrasound unit 2A and 2C in the example of this drawing) is inserted inside the hard tube 5E, and the insertion member 6B is inserted inside the hard tube 5E. Thus, at least one each of the image capture units and the ultrasound units, the hard tube 5D and the insertion member 6D construct a hard endoscope E7. In this way, a single hard endoscope E7 can obtain an image captured image and can also obtain a tomographic image or the like, so that a plurality of image information can be obtained.

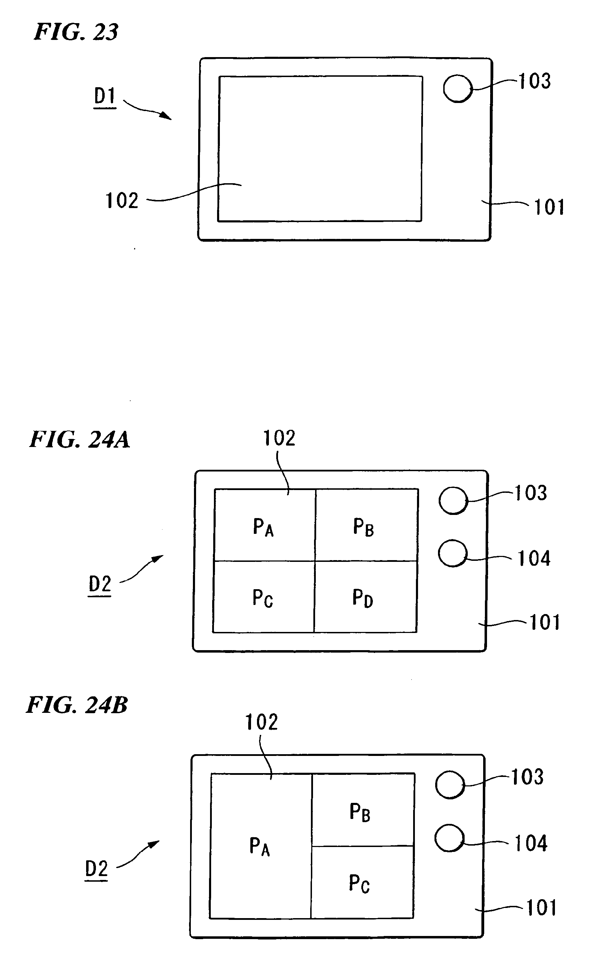

Here, examples of the display apparatuses that are appropriate for use as peripheral devices with the above described hard endoscopes E1 through E6 are shown in FIG. 23, FIG. 24A and FIG. 24B.
FIG. 23 shows a display device D1 with a case 101, in which an LCD (liquid crystal display) or similar display element 102 is provided, that displays an image from the respective observation units provided on the hard endoscopes E1 through E6, and which may be provided integrally to the above described each hard endoscope E1 through E6, or may be provided separately. In this display apparatus D1, although omitted in the drawing, there are appropriately provided a wireless receiving circuit (including an antenna) that receives the wireless signal from the respective observation units, a display element control circuit that performs display control of the display element 102, or a battery or the like that supplies electric power to the respective components inside the display apparatus D1.

Also, in this display apparatus D1, a toggle switch 103 is provided. This toggle switch 103 operates with the display element control circuit to selectively display the image from the respective observation units on the display element 102. In the case where a plurality of observation units is provided, as in the hard endoscope E6 for example, the image from each observation unit can be respectively selectively displayed. Moreover, the images from a plurality of hard endoscopes E1 through E6 can be selectively displayed.

FIG. 24A and FIG, 24B show a display apparatus D2, being a modified example of the display apparatus D, in which the display area of the screen of the display element 102 can be divided into a plurality of areas, each display area respectively being made to correspond to the inherent identifier provided to each observation unit, to enable the display of a plurality of images on a single screen. On this display apparatus D2, a setting button 104 that operates with the display element control circuit is provided. By operating this setting button 104, the display area of the image from the respective observation units can be allocated and made to correspond to the respective identifiers, or the display area lay out can be set and so forth. For example in the case of using four observation units, as shown in FIG. 24A, the display area can be divided into roughly four equal parts, and the image from each observation unit can be respectively allocated to display areas PA through PD to allow them to be simultaneously displayed. Moreover, in cases such as where there is a difference in the level to which the images from each observation unit are required, as shown in FIG. 24B, the display area of an important image (the display area PA in the example of the drawing) can be enlarged to make it easier to see, while the display area of images that are not required as much (the display area PD in the example of the drawing) can be omitted or shrunk to appropriately change the display layout.

In the present embodiment, as an auxiliary unit, only the battery unit has been disclosed. However it is not limited to this. For example, a recording unit provided with a device for recording image information from the image capture unit or ultrasound unit may also be provided as an auxiliary unit.

Also, a hard endoscope has been constructed using a hard tube. However, by using a flexible tube and a flexible insertion member, a flexible endoscope can also be constructed. Also, a tube formed from separate members with a flexible forward side and a hard backward side may also be used

### [Second Embodiment]

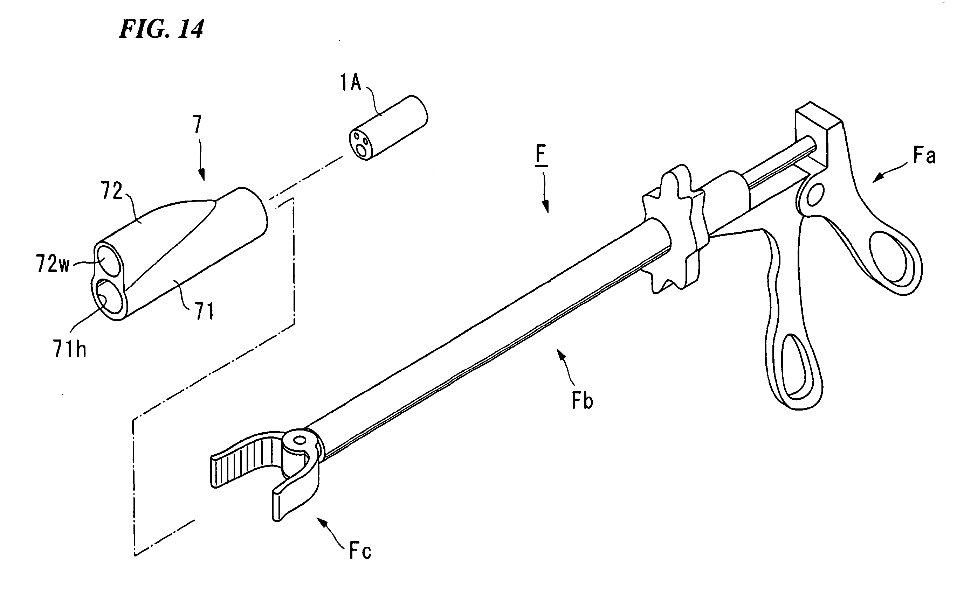

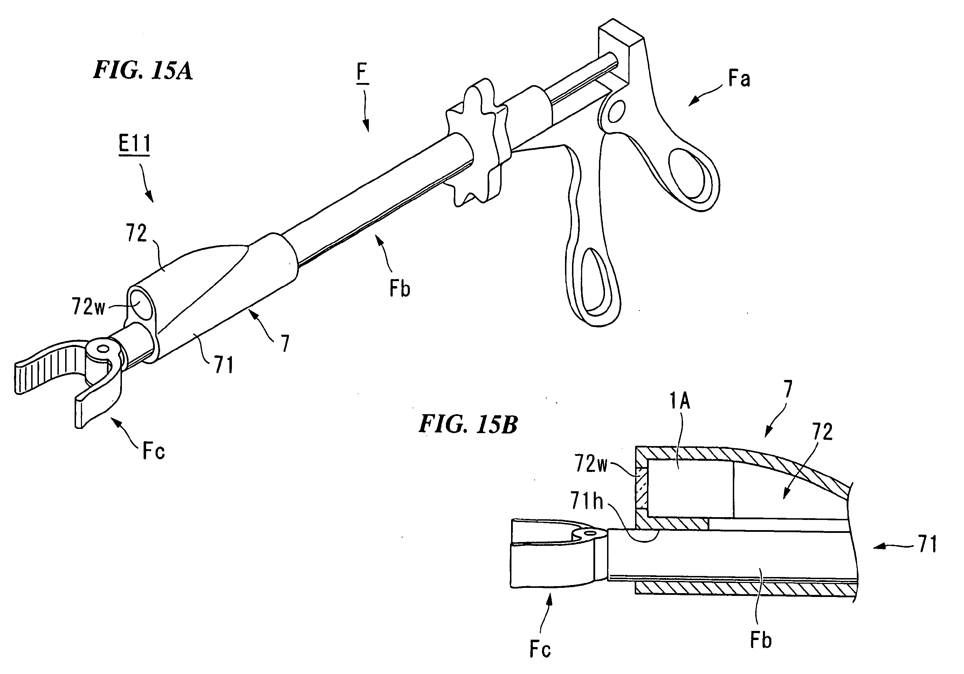

Next, a second embodiment will be described using FIG. 14, FIG. 15A and FIG. 15B. In the present embodiment, an observation unit is made integral with a forceps via a holding device, to show an example of a construction of an observation system where a forceps is made to have the function of an endoscope. In the description below, components that are the same as those in the first embodiment are denoted by the same reference symbols, and detailed description thereof is omitted.

A forceps F used in the present embodiment, as shown in FIG. 14, FIG. 15A and FIG. 15B, is a holding forceps including an operation section Fa on the base end side, a shaft section Fb, and a holding section Fc on the tip end side. To this forceps F an observation unit is attached via a holding member (holding device) 7 to construct an observation system E11. In these drawings, as an observation unit, only the image capture unit 1A is shown as an example. However it is of course possible to change this with the image capture units 1B through 1F as appropriate.

The holding member 7 is provided with a linking section 71 that is linked with the forceps F, and a unit holding section 72 that holds the image capture unit 1A through 1F. In the linking section 71 is formed a through-hole 71h through which the shaft Fb of the forceps F is passed, integrally attaching the linking section 71 to the shaft Fb. Also, in the forward side of the unit holding section 72, a window section 72w is formed so that the observation units 1A through 1F can image-capture an observation image.

By inserting and fixing the image capture unit 1A inside the unit holding section 72 and then linking the linking section 71 to the shaft section Fb, an observation system E11 in which the forceps F and the image capture unit 1A are integrated via the holding member 7 is constructed. As a result, the forceps F can be given the function of an endoscope, so that while it is performing a procedure and so forth on the internal wall of a body cavity, image capture of the procedure site can be performed at close range. Therefore, compared with the case of using an endoscope and forceps separately, convenience can be increased, and the procedure can be performed more accurately.

In the present embodiment, an example has been described with a single image capture unit only as the observation unit. However it is also possible to use a combination of a plurality of units. That is to say, as with the hard endoscope described in the above first embodiment, an image capture unit and an ultrasound unit may be combined, and an auxiliary unit may be used. In this case, the structure of the holding member is modified to suit the units used. For example, the size of the unit holding section and the formation position of the window section may be modified to provide a light source and the like to illuminate an observed portion. Also, with the observation system E11 it is of course possible to use the display apparatuses D1 and D2 shown in the above described first embodiment.

### [Third exemplary Embodiment]

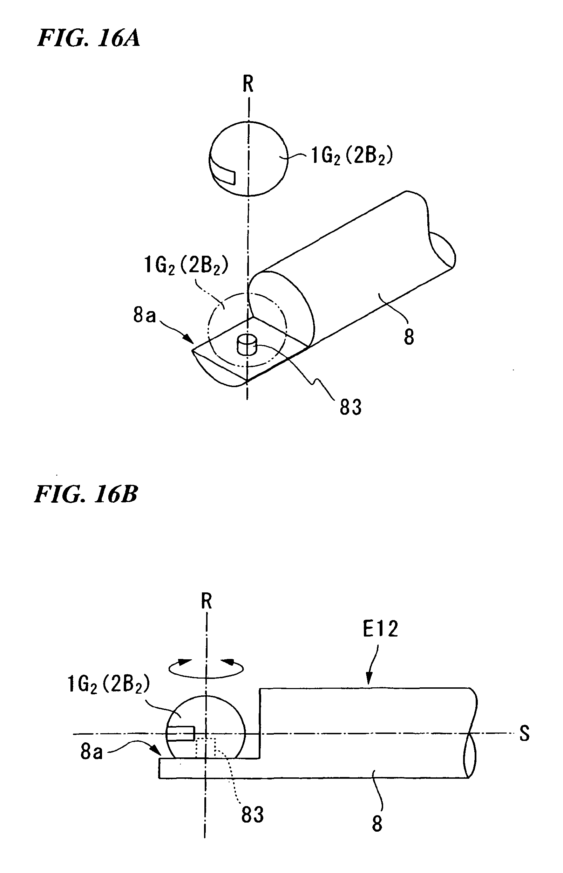

Next, a third exemplary embodiment will be described using FIG. 16A through FIG. 18B. In the present exemplary embodiment, an observation unit represented by an image capture unit or an ultrasound unit is rotated to show an example of a construction where the direction of image information obtaining can be changed. That is to say, this is a modification example of the hard endoscope E4 in the above first embodiment.

A hard endoscope (observation system) E12 according to the present exemplary embodiment, as shown in FIG. 16A and FIG. 16B, is constructed with an image capture unit 1G2 (alternatively an ultrasound unit 2B2 may be used instead), and a holding member 8 that rotatably holds the image capture unit 1G2 at the tip end side. The image capture unit 1 G2 and the ultrasound unit 2B2 are respective modified examples of the above described image capture unit 1G and the ultrasound unit 2B, which are able to obtain image information in the sideways direction with respect to their axis of rotation, and have an engagement hole 10h that is engaged with a rotation shaft 83 (described later) of the holding member 8 formed therein. The push-button switch 15b is omitted.

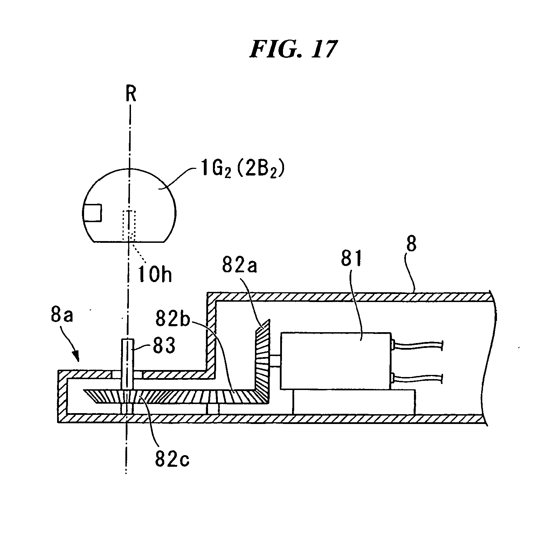

The holding member 8 includes a long pole-shaped hard member, at the tip end side of which is formed a unit holding section 8a for holding the image capture unit 1G2. Inside this holding member 8, as shown in FIG. 17, a motor (rotation driving device) 81 with a bevel gear 82a, a bevel gear 82b that engages with the bevel gear 82a, and the rotation shaft 83, which is integrally provided with a bevel gear 82c that engages with the bevel gear 82b, are housed. The rotation shaft 83 projects toward the unit holding section 8a. Although not shown in the drawing, inside the holding member 8, a battery may be fitted to supply electric power to the motor 81.

In this hard endoscope E12, by attaching the image capture unit 1G2 to the rotation shaft 83 and appropriately rotating the motor 81, the image capture unit G2 is rotation driven about the rotation axis R in the drawing allowing the image information obtaining direction to be varied within a plane S in the drawing. Therefore, even if the holding member 8 is fixed, image information can be obtained from various directions.

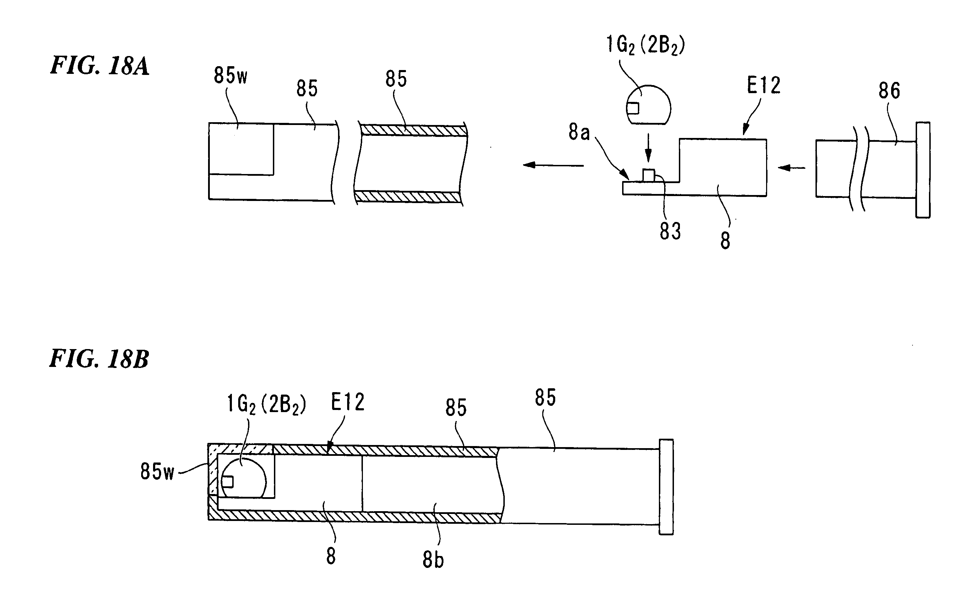

This hard endoscope E12, as shown in FIG. 18A and FIG. 18B, may also be used inserted inside the flexible tube 85. In this example, the hard endoscope E12 is inserted inside the flexible tube 85 with a window section 85w formed in its tip end side, and is covered with the flexible lid section 86. As a result, a flexible endoscope in which bodily fluid such as blood does not become attached to the image capture unit can be constructed.

### [Fourth exemplary Embodiment]

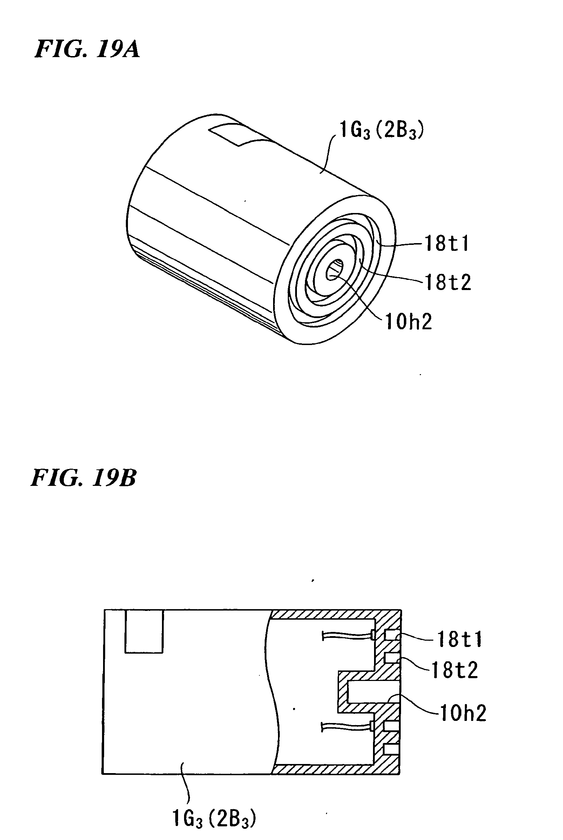

Next, a fourth exemplary embodiment will be described using FIG. 19A through FIG. 22C. In the present exemplary embodiment, an observation unit represented by an image capture unit or an ultrasound unit is rotated to show an example of a construction where the direction of image information obtaining can be changed. That is to say, this is a modification example of the hard endoscope E4 in the above first embodiment.

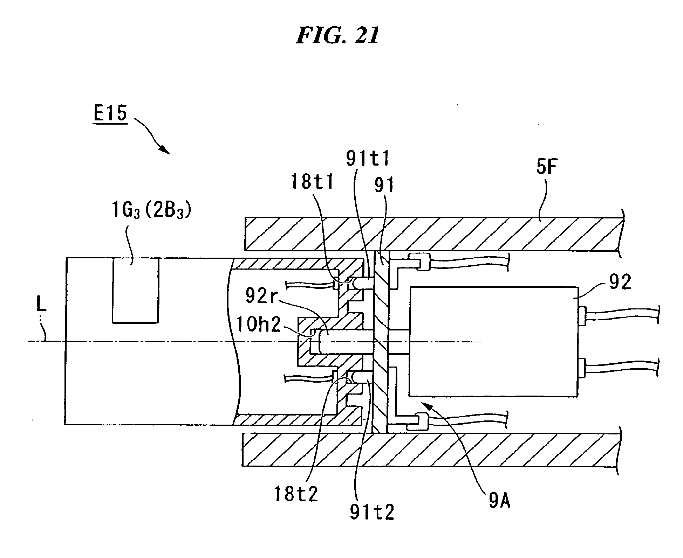

As shown in FIG. 21, a hard endoscope (observation system) E15 according to the present exemplary embodiment is constructed from a hard tube (holding device) 5F of a cylinder shape with an open mouth on the tip end side, an image capture unit 1G3 (alternatively an ultrasound unit 2B3 may be used instead), and a holding unit (holding device) 9A that is integrally provided with the hard tube 5F and that holds the image capture unit 1G3 allowing it to rotate around the axis L.

As shown in FIG. 19A and FIG. 19B, the image capture unit 1G3 (alternatively the ultrasound unit 2B3) are respectively modified examples of the above described image capture unit 1G and ultrasound unit 2B, which are able to obtain image information in the side direction with respect to their axes of rotation. In the backward direction thereof, an engagement hole 10h2 that is engaged with a rotation shaft 92r (described later) of a motor (rotation driving device) 92, and ring-shaped groove terminals 18t1 and 18t2, are formed concentrically in a circle shape. The push-button switch 15b is omitted. The terminals 18t1 and 18t2 are modified examples of the terminal 18t shown in the above described image capture unit 1G and ultrasound unit 2B, sliding with, while being electrically connected respectively to, terminals 91t1 and 91t2 of the holding unit 9A for holding the auxiliary power supply circuit 18 (not shown in the drawing here).

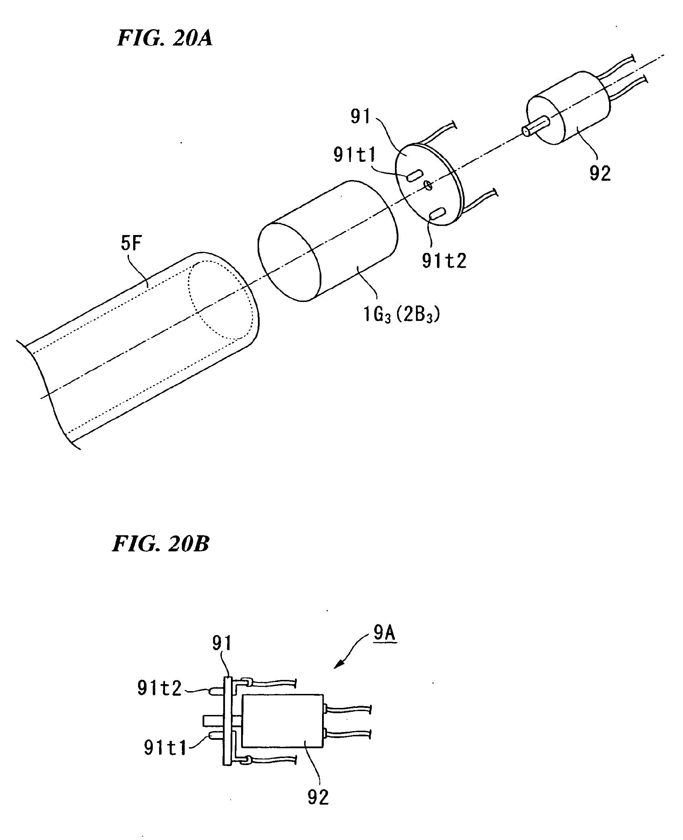

The holding unit 9A, as shown in FIG. 20A and FIG. 20B, is constructed from a linking plate 91 provided with the terminals 91t1 and 91t2, and a motor 92. The linking plate 91 has an outer diameter substantially equal to the inner diameter of the hard tube 5F, and when inserted inside the hard tube it is integrally fixed with the hard tube 5F. The terminals 91t1 and 91t2 are respectively pin shaped, project to the front of the linking plate 91, and are respectively electrically connected to the terminals 18t1 and 18t2 of the image capture unit 1G3. Also, the motor 92 is integrally provided on the backward end side of the linking plate 91, with the rotation shaft 92r passing through the linking plate 91 and projecting out in the forward direction. The terminals 91t1 and 91t2 and the motor 92 are connected to a battery external to the image capture unit and not shown in the drawing.

To assemble this hard endoscope E15, the holder unit 9A is fixed inside the hard tube 5A and the engagement hole 10h2 is engaged with the rotation shaft 92r of the motor 92 to thereby attach the image capture unit 1G3. As a result, the terminals 91t1 and 91t2 are inserted inside the terminals 18t1 and 18t2, electrically connecting them. In this condition, when the motor 92 is rotated, the image capture unit 1G3 is rotated along with the rotation shaft 92r around the axis L, and the image information in front of the hard endoscope E15 can be obtained around almost its entire circumference.

Now, even if the image capture unit 1G3 rotates, the terminals 91t1 and 91t2 and the terminals 18t1 and 18t2 slide against each other while being electrically connected, so that electric power from an external battery can be supplied to the image capture unit 1G3. Accordingly, the hard endoscope E15 can be of a simple construction, while the image capture unit 1G3 can be driven by electric power from the outside, enabling it to be used for a long time.

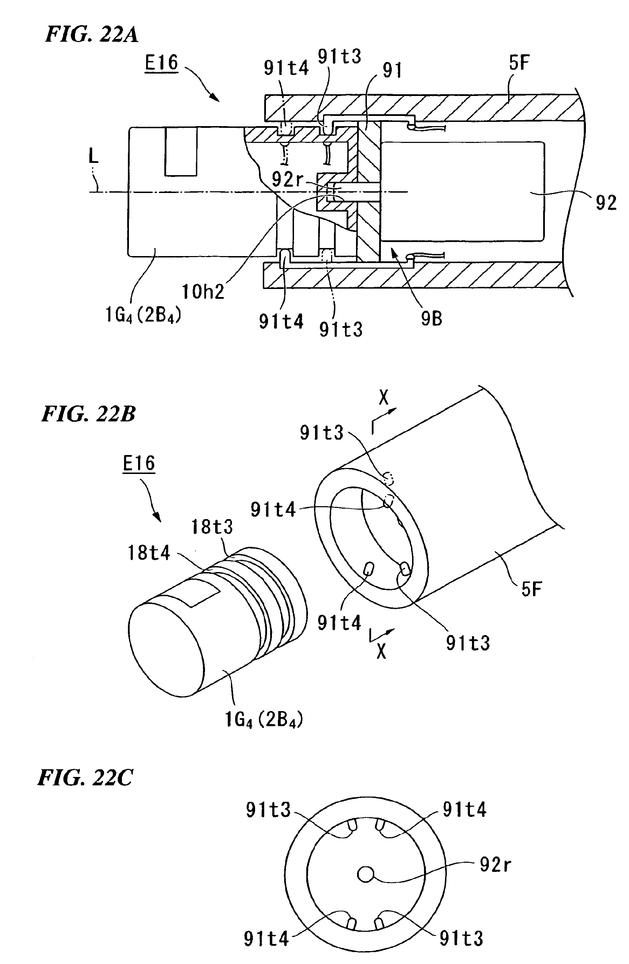

In FIG. 22A through FIG. 22C, a hard endoscope (observation system) E16 is shown as a modified example of the above described hard endoscope E15. This hard endoscope E16 is constructed from a hard tube 5F, an image capture 1G4 (alternatively an ultrasound unit 2B4 may be used instead), and a holding unit (holding unit) 9B that is integrally provided with the hard tube 5F and that holds the image capture unit 1G4 allowing it to rotate around the axis L.

The image capture unit 1G4 and the ultrasound unit 2B4 are different compared to the image capture unit 1G3 and the ultrasound unit 2B3 in that the terminals are formed in different positions. That is to say, as shown in FIG. 22A and FIG. 22B, in place of the terminals 18t1 and 18t2 formed on the backward side, terminals 18t3 and 18t4 are formed in a groove shape around the entire circumference on the outside face side. In addition, the holding unit 9B has terminals formed in a different position compared with the holding unit 9A. That is to say, the terminals 91t3 and 91t4 of the holding unit 9B respectively correspond to the terminals 18t3 and 18t4, being formed so as to project towards the inner circumference side of the hard tube 5F on the forward side of the linking plate 91.

In this hard endoscope E16, even if the image capture unit 1G4 rotates, the terminals 91t3 and 91t4 and the terminals 18t3 and 18t4 slide against each other while being electrically connected, so that electric power from a battery external to the image capture unit can be supplied to the image capture unit 1G4. Accordingly, the hard endoscope E16 can be of a simple construction, while the image capture unit 1G4 can be driven by electric power from the outside, enabling it to be used for a long time.

It is of course possible to use the display apparatuses D1 and D2 with the hard endoscopes E15 and E 16 shown in the above described first embodiment.

In the above, preferred embodiments of the present invention have been described. However, the present invention is not limited by these. Without departing from the scope of the present invention, additions, omissions and replacements and other changes can be made to its construction. The present invention is not to be limited by the above descriptions and is to be limited only by the scope of the appended claims.

### INDUSTRIAL APPLICABILITY

An observation system according to the present invention may preferably find use not only in medicine as described above, but also in industrial applications.

## Claims

1. An observation system (E1, E2, ..., E7, E11) comprising:
an observation unit (1A, 1B, 2A, 2B) having an image information obtaining device that is adapted to obtain image information of an observation target, an image signal processing device (13) that is adapted to perform image signal processing of image information obtained by the image information obtaining device, and a transmission device (14) that is adapted to transmit an image signal from after the image signal processing has been carried out, to the outside; and
a holding device (5A, 5B) that is adapted to detachably hold one or a plurality of the observation units (1A, 1B, 2A, 2B), wherein the holding device (5A, 5B) is a cylinder shaped body in which a window section (51) is formed in at least one portion, and one or a plurality of the observation units (1A, 1B, 2A, 2B) are held inside the cylinder shaped body and are adapted to obtain image information of the observation target via the window section (51), **characterized in that**
the observation unit (1A, 1B, 2A, 2B) is adapted to be inserted into the holding device (5A, 5B) along an axial direction of the cylinder shaped body.

2. The observation system (E1, E2, ..., E7, E11) according to claim 1, wherein the transmission device (14) is a wireless transmitting and receiving device that is adapted to transmit the image signal wirelessly to a peripheral device (D1, D2), and that is adapted to receive various kinds of signals wirelessly from the peripheral device (D1, D2).

3. The observation system (E1, E2, ..., E7, E11) according to claim 1, wherein the observation unit (1A, 1B, 2A, 2B) is an image capture unit provided with an image capture element (12A, 12B) as the image information obtaining device that is adapted to image-capture an observed image of the observation target.

4. The observation system (E1, E2, ..., E7, E11) according to claim 1, wherein the observation unit (2A, 2B) is an ultrasound unit provided with an oscillator (21A, 21B) as the image information obtaining device that is adapted to emit ultrasonic waves toward an observed portion of the observation target and is adapted to receive ultrasonic waves reflected from the observed portion.

5. The observation system (E1, E2, ..., E7, E11) according to claim 1, wherein the holding device (5B) is provided with a rotation driving device (63m) that is adapted to rotate the observation unit (2B) with respect to the holding device (5B) to vary an image information obtaining direction.

6. The observation system (E1, E2, ..., E7, E11) according to claim 1, further comprising an auxiliary unit (3) that is adapted to be held together with the observation unit (1A, 1B, 2A, 2B), by the holding device (5A, 5B), and that is adapted to supply electric power to the observation unit (1A, 1B, 2A, 2B).

## Patentansprüche

1. Beobachtungssystem (E1, E2, ..., E7, E11) mit:
einer Beobachtungseinheit (1A, 1B, 2A, 2B) mit einer Bildinformations-Erfassungsvorrichtung, die Bildinformation eines Beobachtungsziels zu erfassen vermag, einer Bildsignal-Verarbeitungsvorrichtung (13), die eine Bildsignalverarbeitung von über die Bildinformations-Erfassungsvorrichtung erhaltener Bildinformation auszuführen vermag, und einer Übertragungsvorrichtung (14), die ein Bildsignal nach erfolgter Bildsignalverarbeitung nach außen zu übertragen vermag; und
einer Haltevorrichtung (5A, 5B), die eine oder mehrere Beobachtungseinheit(en) (1A, 1B, 2A, 2B) lösbar zu halten vermag, wobei die Haltevorrichtung (5A, 5B) ein zylinderförmiger Körper ist, bei dem in mindestens einem Teil ein Fensterabschnitt (51) ausgebildet ist, und eine oder mehrere Beobachtungseinheit(en) (1A, 1B, 2A, 2B) in dem zylinderförmigen Körper untergebracht sind und Bildinformation des Beobachtungsziels über den Fensterabschnitt (51) zu erfassen vermögen, **dadurch gekennzeichnet, dass**
die Beobachtungseinheit (1A, 1B, 2A, 2B) entlang einer axialen Richtung des zylinderförmigen Körpers in die Haltevorrichtung (5A, 5B) eingesetzt zu werden vermag.

2. Beobachtungssystem (E1, E2, ..., E7, E11) nach Anspruch 1, wobei die Übertragungsvorrichtung (14) eine drahtlose Übertragungs- und Empfangsvorrichtung ist, die das Bildsignal drahtlos an ein Endgerät (D1, D2) zu übertragen vermag, und die verschiedene Arten von Signalen drahtlos von dem Endgerät (D1, D2) zu empfangen vermag.

3. Beobachtungssystem (E1, E2, ..., E7, E11) nach Anspruch 1, wobei die Beobachtungseinheit (1A, 1B, 2A, 2B) eine Bildaufnahmeeinheit ist, die mit einem Bildaufnahmeelement (12A, 12B) als der Bildinformations-Erfassungsvorichtung versehen ist, welche eine Bildaufnahme eines beobachteten Bildes des Beobachtungsziels vorzunehmen vermag.

4. Beobachtungssystem (E1, E2, ..., E7, E11) nach Anspruch 1, wobei die Beobachtungseinheit (2A, 2B) eine mit einem Oszillator (21A, 21B) als der Bildinformations-Erfassungsvorrichtung versehene Ultraschalleinheit ist, welche Ultraschallwellen zu einem beobachteten Abschnitt des Beobachtungsziels zu emittieren vermag und von dem beobachteten Abschnitt reflektierte Ultraschallwellen zu empfangen vermag.

5. Beobachtungssystem (E1, E2, ..., E7, E11) nach Anspruch 1, wobei die Haltevorrichtung (5B) mit einer Drehantriebsvorrichtung (63m) versehen ist, welche die Beobachtungseinheit (2B) in Bezug auf die Haltevorrichtung (5B) zu drehen vermag, um eine Bildinformation-Erfassungsrichtung zu variieren.

6. Beobachtungssystem (E1, E2, ..., E7, E11) nach Anspruch 1, ferner mit einer Hilfseinheit (3), die mit der Beobachtungseinheit (1A, 1B, 2A, 2B) durch die Haltevorrichtung (5A, 5B) zusammengehalten zu werden vermag, und die der Beobachtungseinheit (1A, 1B, 2A, 2B) elektrische Energie zu liefern vermag.

## Revendications

1. Système d'observation (E1, E2, ..., E7, E11) comprenant :
une unité d'observation (1A, 1B, 2A, 2B) comportant un dispositif d'obtention d'informations d'image qui est adapté pour obtenir des informations d'image d'une cible d'observation, un dispositif de traitement de signal d'image (13) qui est adapté pour réaliser un traitement de signal d'image d'informations d'image obtenues par le dispositif d'obtention d'informations d'image, et un dispositif de transmission (14) qui est adapté pour transmettre un signal d'image après que le traitement de signal d'image ait été réalisé, vers l'extérieur ; et
un dispositif de maintien (5A, 5B) qui est adapté pour maintenir de manière détachée une ou une pluralité d'unités d'observation (1A, 1B, 2A, 2B), dans lequel le dispositif de maintien (5A, 5B) est un corps de forme cylindrique dans lequel une section de fenêtre (51) est formée dans au moins une partie, et une ou une pluralité d'unités d'observation (1A, 1B, 2A, 2B) sont maintenues à l'intérieur du corps de forme cylindrique et sont adaptées pour obtenir des informations d'image de la cible d'observation via la section de fenêtre (51), **caractérisé en ce que**
l'unité d'observation (1A, 1B, 2A, 2B) est adaptée pour être insérée dans le dispositif de maintien (5A, 5B) le long d'une direction axiale du corps de forme cylindrique.

2. Système d'observation (E1, E2, ..., E7, E11) selon la revendication 1, dans lequel le dispositif de transmission (14) est un dispositif de transmission et de réception sans fil qui est adapté pour transmettre le signal d'image sans fil vers un dispositif périphérique (D1, D2), et qui est adapté pour recevoir divers types de signaux sans fil depuis le dispositif périphérique (D1, D2).

3. Système d'observation (E1, E2, ..., E7, E11) selon la revendication 1, dans lequel l'unité d'observation (1A, 1B, 2A, 2B) est une unité de capture d'image munie d'un élément de capture d'image (12A, 12B) en tant que dispositif d'obtention d'informations d'image qui est adapté pour saisir en image une image observée de la cible d'observation.

4. Système d'observation (E1, E2, ..., E7, E11) selon la revendication 1, dans lequel l'unité d'observation (2A, 2B) est une unité ultrasonore munie d'un oscillateur (21A, 21B) en tant que dispositif d'obtention d'informations d'image qui est adapté pour émettre des ondes ultrasonores vers une partie observée de la cible d'observation et est adapté pour recevoir des ondes ultrasonores réfléchies depuis la partie observée.

5. Système d'observation (E1, E2, ..., E7, E11) selon la revendication 1, dans lequel le dispositif de maintien (5B) est muni d'un dispositif d'entraînement rotationnel (63m) qui est adapté pour faire tourner l'unité d'observation (2B) par rapport au dispositif de maintien (5B) pour faire varier un sens d'obtention d'informations d'image.

6. Système d'observation (E1, E2, ..., E7, E11) selon la revendication 1, comprenant en outre une unité auxiliaire (3) qui est adaptée pour être maintenue conjointement avec l'unité d'observation (1A, 1B, 2A, 2B), par le dispositif de maintien (5A, 5B) et qui est adaptée pour délivrer la puissance électrique vers l'unité d'observation (1A, 1B, 2A, 2B).
